# EUROPEAN PATENT SPECIFICATION

(11) **EP 2 008 641 B1**
(45) Date of publication and mention of the grant of the patent: **07.05.2014**
(21) Application number: 07738811.4
(22) Date of filing: 16.03.2007
(51) Int. Cl.: A61K 8/25, C09C 1/00

(54) **COSMETIC PREPARATION CONTAINING FLAKY GLASS**
BLÄTTCHENFÖRMIGES GLAS ENTHALTENDE KOSMETISCHE ZUBEREITUNG
PRÉPARATION COSMÉTIQUE CONTENANT DU VERRE LAMELLAIRE

(30) Priority: 20.03.2006 JP 2006077128
(43) Date of publication of application: 31.12.2008
(73) Proprietor: Nippon Sheet Glass Company, Limited, Tokyo 108-6321 (JP)
(72) Inventor: YAGYU, Tomohiro, Tokyo 108-6321 (JP); YANAGASE, Shigeru, Tokyo 108-6321 (JP); ISHIBE, Hisao, Tokyo 108-6321 (JP)
(74) Representative: Hart-Davis, Jason
(86) International application number: PCT/JP2007/055366
(87) International publication number: WO 2007/119395

(56) References cited:
- WO-A1-2005/028566
- WO-A1-2005/028566
- JP-A- 2005 187 782
- US-A1- 2005 113 485
- DATABASE WPI Week 200224 Thomson Scientific, London, GB; AN 2002-188724 XP002681627, & WO 02/10291 A1 (NIPPON SHEET GLASS CO LTD) 7 February 2002 (2002-02-07)
- DATABASE WPI Week 200556 Thomson Scientific, London, GB; AN 2005-544792 XP002681628, & JP 2005 187782 A (NIPPON SHEET GLASS CO LTD) 14 July 2005 (2005-07-14)

## Description

### Technical Field

The present invention relates to cosmetics, and in particular, to a cosmetic containing glass flakes as an extender pigment.

### Background Art

In makeup cosmetics such as face powder, powder foundation, cheek rouge and eye shadow, natural flaky extender pigments such as talc, mica and sericite are mainly used. In particular, mica, sericite and silica flakes (flaky silica) are transparent and soft to the touch, and are easily removed when they are processed into pressed cake type cosmetics. Thus, they are used widely.

However, since mica and sericite are natural products, they contain impurities such as iron. Thus, they tend to darken when oil is added thereto, which causes a problem that good color development cannot be obtained.

Furthermore, mica and sericite have other problems that those having high aspect ratios are less commonly available, and that they are not sufficient in adhesion to the skin and smoothness.

Meanwhile, synthetic mica and amorphous flaky silica (see, for example, JP 06(1994)-87720 A), and crystalline flaky silica (see, for example, JP 04(1992)-145011 A) also are used instead of the natural products.
However, these materials suffer from such problems that those having high aspect ratios are less commonly available, and that they do not have sufficient strength and thus are crushed when they are mixed with other powder materials. The above-mentioned amorphous flaky silica and crystalline flaky silica are obtained by applying a silica coating on a substrate such as a stainless steel sheet and a steel sheet, drying it, and then peeling off the coating film from the substrate. Therefore, asperities on the surface of the substrate are transferred to flaky silica, resulting in a product with the asperities being formed on one surface thereof (the surface that has come into contact with the substrate during production thereof), which causes surface reflection. As a result, it is difficult to maintain the transparency of the glass flakes, and the obtained glass flakes are too glossy.

Furthermore, makeup cosmetics containing irregular-shaped silica gel are also proposed. However, since silica gel is a porous material, cosmetics containing such silica gel tend to absorb moisture of the skin, resulting in rough feeling and poor spreading on the skin.

On the other hand, mica and synthetic mica are too glossy. Thus, they also have a problem that a natural makeup effect cannot be obtained due to their excessive gloss. That is, a natural makeup finish like a natural skin tone hardly can be obtained.

WO 2005/028566 A1 shows a black bright pigment which comprises a base material in the form of flakes having an average thickness of 0.1 to 8.0 µm, an average particle diameter of 1 to 800 µm and an aspect ratio of 3 to 500 and, formed on the surface thereof, a coated thin film of tri-iron tetroxide or a lower order titanium oxide, wherein the thin film preferably has a thickness of 10 nm to 1.0 µm and preferably exhibits a brightness (L15 value) of 40 or higher and the black bright pigment exhibits a black color having brightness and a high contrast ratio for a cosmetic or the like.

According to WO 02/10291 A1, glass flakes to be blended in cosmetics, which can prevent the elution of boron, heavy metals and other components and are not yet worsened in the characteristics as pigments and characteristics as cosmetics, are produced by coating the surface with titania and then forming a coating film for preventing the elution of glassy components by using at least one metal oxide selected from the group consisting of silica, alumina, zirconia, zinc oxide and cerium oxide.

According to JP20030434489 A, a pearl tone glossy pigment for cosmetics contains a scale-like glass base material of A-glass composition or E-glass composition, surface-coated with iron oxide and/or titanium dioxide as a scale-like glass base material with an average thickness of 0.1 - 20 µm and an average particle diameter of 5 - 2000 µm.

### Disclosure of Invention

It is an object of the present invention to solve the above-mentioned problems and to provide a makeup cosmetic that is smooth and good to the tough, is excellent in transparency and adhesion to the skin, and exhibits a natural finish with moderate gloss, namely a so-called natural skin finish.

In order to achieve the above object, the cosmetic of the present invention is defined in claim 1 and is a cosmetic containing glass flakes, in which the glass flakes have a composition including at least 52 mass% of silicon dioxide and 5 mass% or less of alkali metal oxides, the glass flakes have an average thickness of 0.1 to 1.0 µm, an average particle diameter of 1 to 100 µm, and an average aspect ratio of 10 or greater. The average aspect ratio is obtained by dividing the average particle diameter by the average thickness. Further, in this cosmetic, a particle diameter distribution index of the glass flakes is 5.0 or less. The particle diameter distribution index is obtained by dividing D90 by D10, where in the particle size distribution of the glass flakes, D10 is a particle diameter at which the cumulative mass distribution of particle diameters reaches 10% when counted from the smaller side, and D90 is a particle diameter at which the cumulative mass distribution of particle diameters reaches 90% when counted from the smaller side.

It should be noted that a particle size distribution is an index indicating the sizes (diameters) of particles to be measured and the relative amounts of the particles sorted into these sizes. In the present description, the particle size distribution is measured based on a laser diffraction/scattering method. The laser diffraction/scattering method is a method for measuring particle size by employing the fact that particles of different diameters have their own patterns of scattering light amounts when they are irradiated with a laser beam. According to this method, the average particle diameter and the distribution of the particles can be obtained. Mass is used as a measure of an amount of particles. The definitions of D10 and D90 are as described above. More specifically, D90 is defined as a particle diameter at which the cumulative mass distribution (cumulative mass percent distribution) of particle diameters reaches 90% when counted from the smaller side. In other words, D90 indicates that, among all the particles, 90 mass% of particles have smaller diameters than the diameter indicated as D90. Likewise, D10 is defined as a particle diameter at which the cumulative mass distribution of particle diameters reaches 10%. D10 indicates that, among all the particles, 10 mass% of particles have smaller diameters than the diameter indicated as D10. The particle diameter distribution index represented by D90/D10 can be regarded as an index indicating a range of particle size distribution, that is, variations in particle size. The range of particle size distribution becomes wider as the particle diameter distribution index increases, and the range becomes narrower (uniformity of particle size becomes higher) as the index decreases.

In the present description, an average particle diameter means a particle diameter at which the cumulative mass distribution of particle diameters reaches 50% (D50).

The cosmetic of the present invention contains, as an extender pigment, glass flakes having a high strength, exhibiting translucent dullness-free color, and having a high aspect ratio. Therefore, cosmetics produced by adding coloring pigments and others to these glass flakes can achieve a very high transparency. In addition, since these glass flakes have sufficient strength, they are hardly crushed when they are mixed with other powder materials. Thus, the effect of these glass flakes are not impaired when they are mixed in cosmetics.

Furthermore, the cosmetic of the present invention contains thin glass flakes with a high aspect ratio. Thus, these glass flakes make it possible to obtain a cosmetic that is excellent in adhesion to the skin, and exhibits a natural finish with moderate gloss, so-called a natural skin finish, compared to a cosmetic containing, as an extender pigment, mica, sericite, synthetic mica, or flaky silica only.

Moreover, the particle diameter distribution index (D90/D10) of these glass flakes is 5.0 or less, which means that the uniformity of the particle diameters is high. Thus, it is possible to realize a cosmetic being smooth and good to the touch and good in spreading on the skin as well. That is, a cosmetic having a good feeling of use can be obtained. In addition, the use of these glass flakes with a high uniformity of particle diameters allows a cosmetic containing them to be removed easily when washing the face. Thus, the cosmetic of the present invention that contains glass flakes with a high uniformity of particle diameters can achieve a good quality product.

As described above, according to the present invention, it is possible to obtain a cosmetic that is smooth and good to the touch, is excellent in transparency, color development property and adhesion to the skin, and exhibits a natural finish with moderate gloss, namely a so-called natural skin finish.

### Best Mode for Carrying Out the Invention

An embodiment of the cosmetic of the present invention will be described below.

The cosmetic of the present embodiment contains glass flakes as a component thereof. The average thickness of these glass flakes is in a range of 0.1 to 1.0 µm. The average thickness of 0.1 µm or more can provide the glass flakes with enough strength to keep from being crushed during manufacturing of the cosmetic (mixing with other components). The average thickness of 1.0 µm or less also can suppress a gritty feeling when they are mixed in the cosmetic. In order to produce a cosmetic with a better feeling, the average thickness of the glass flakes is preferably 0.8 µm or less, and more preferably 0.6 µm or less. The average particle diameter of the glass flakes is in a range of 1 to 100 µm, preferably in a range of 1 to 50 µm, and more preferably in a range of 5 to 30 µm. The average particle diameter within these ranges makes it possible to suppress a gritty feeling when they are mixed in the cosmetic, thereby achieving a good adhesion to the skin and a high smoothness on the skin. The average aspect ratio (a ratio of an average particle diameter to an average thickness) of these glass flakes is at least 10, and more preferably at least 20. Although there is no particular limitation on the upper limit of the average aspect ratio, it can be 500 or less, for example. When glass flakes having the aspect ratio as described above are mixed in a cosmetic, the cosmetic can exhibit a good adhesion to the skin and a good and smooth spreading on the skin.

The particle diameter distribution index of the glass flakes to be used for the cosmetic of the present embodiment is 5.0 or less. Thus, by using the glass flakes with a small particle diameter distribution index (with a high uniformity of particle diameters), it is possible to obtain a cosmetic having a good feeling of use, namely, a cosmetic that is smooth and good to the touch and is good in spreading on the skin. In addition, by using the glass flakes with a high uniformity of particle diameters, it is possible to obtain a cosmetic that can exhibit a beautiful finish and further can be removed easily when washing the face. In order to improve the quality of the cosmetic, it is preferable to use glass flakes having a particle diameter distribution index of 4.0 or less. The properties of the cosmetic improve as the uniformity of particle diameters increases. Therefore, it is desirable that the particle diameter distribution index is as small as possible, and the lower limit thereof is not particularly limited.

The glass flakes to be used in the present embodiment can be produced using, for example, a so-called blowing method as disclosed in JP 41(1966)-17148 B and JP 45(1970)-3541 B, and a so-called rotary method as disclosed in JP 59(1984)-21533 A and JP 2(1990)-503669 T.

In the blowing method, a nozzle is put in a liquid tank containing molten glass, air is blown through the nozzle to inflate the molten glass into a so-called balloon, and the balloon is drawn by rollers. Thus, glass flakes are obtained. In the rotary method, molten glass is poured continuously into a rapidly rotating flat plate or bowl, and the molten glass is stretched over the rim of the plate or the bowl. Thus, glass flakes are obtained.

The glass flakes to be used in the present embodiment should be substantially free from so-called heavy metals such as lead and arsenic as components of the composition. The content of alkali metals such as sodium and potassium also should be low. The content of alkali metals is 5 mass% or less when expressed in terms of alkali metal oxides, preferably 3 mass% or less, and more preferably 1 mass% or less. The glass flakes may be free from alkali metals (i.e., the content thereof may be 0 mass%). In the present embodiment, the content of silicon dioxide as a component of the composition of the glass flakes is at least 52 mass%. This type of glass is E glass or boron-free E glass as disclosed in JP 2002-226732 A and the like. As shown in Table 1, E glass contains 52 to 56 mass% of silicon dioxide (SiO₂) and 0 to 0.8 mass% of alkali metal oxides. Boron-free E glass contains 55 to 67 mass% of silicon dioxide (SiO₂) and 0 to 5 mass% of alkali metal oxides.

**Table 1**

| (Unit: mass%) | | |
|---|---|---|
| Component | E glass | Boron-free E glass |
| SiO₂ | 62 to 56 | 55 to 67 |
| Al₂O₃ | 12 to 16 | 5 to 15 |
| CaO | 16 to 25 | 17 to 25 |
| MgO | 0 to 6 | 0 to 6 |
| Na₂O+K₂O | 0 to 0.8 | 0 to 5 |
| B₂O₃ | 5 to 13 | - |
| TiO₂ | - | 0 to 2 |
| F₂ | 0 to 0.5 | - |

The amount of glass flakes to be mixed in the cosmetic of the present embodiment varies depending on the type of a cosmetic to be obtained. For example, the glass flakes are used in an amount of 5 to 80 mass% of the total amount of extender pigments, and they are used preferably in an amount of 10 to 70 mass% in particular. "The amount of glass flakes to be mixed" here means the amount of glass flakes to be mixed as an extender pigment. It does not include the amount of glass flakes contained, as a luster pigment coated with titanium oxide, iron oxide, or the like, for example, in the cosmetic.

The surface of a glass flake is inherently hydrophilic, and has less affinity for oils to be added during manufacturing a cosmetic. The glass flake has an affinity for oils, with the surface thereof coated with a hydrophobizing agent. In this case, the entire surface of the glass flake may be coated with the hydrophobizing agent, or a part of the surface may be coated.

Examples of the hydrophobizing agents that can be used for this coating treatment include, as components usually to be used for surface treatment of cosmetic pigments: silicone compounds such as methylhydrogenpolysiloxane, reactive alkylpolysiloxane, high-viscosity silicone oil, and silicone resin; surfactants such as an anion activator and a cation activator; polymer compounds such as a silane coupling agent, a titania coupling agent, nylon, polymethylmethacrylate, polyethylene, fluororesin, and polyamino acid; hydrogenated lecithin; acylating collagen; metallic soap; lipophilic wax; and polyhydric alcohol ester. These hydrophobizing agents may be used in combination.

Examples of coating treatment methods using hydrophobizing agents include a liquid spraying method, and a liquid dipping method, as illustrated in JP 60(1985)-69011 A, JP 60(1985)-184571 A, JP 58(1983)-72512 A, JP 61(1986)-73775 A, JP 61(1986)-17667 A, and others. The adhesion amount of the hydrophobizing agent is normally 0.01 to 5 mass% per mass of glass flakes. As a result of the coating treatment, a cosmetic with a better feeling can be obtained.

In addition to the above-mentioned glass flakes, other components that are used usually for cosmetics can be mixed suitably in the cosmetic of the present embodiment as required. Examples of the other components include inorganic powders, organic powders, pigments, dyes, oil components, organic solvents, resins, and plasticizers. Examples of the inorganic powders include talc, kaolin, mica, sericite, other types of micas, magnesium carbonate, calcium carbonate, aluminum silicate, magnesium silicate, and clays.

Examples of the organic powders include powders such as nylon powder, polyethylene powder, polystyrene powder, epoxy powder, acrylic powder, and plastic beads made of nylon, acryl, or the like.

Examples of the pigments include: inorganic white pigments such as microcrystalline cellulose, titanium oxide, zinc oxide, and zirconium oxide; inorganic red pigments such as iron oxide, and iron titanate; inorganic yellow pigments such as yellow iron oxide, and yellow ocher; inorganic black pigments such as black iron oxide, and carbon black; inorganic green pigments such as chromium oxide, chromium hydroxide, and cobalt titanate; inorganic blue pigments such as iron blue; pearl luster pigments such as glass flakes coated with titanium oxide and iron oxide, silica, titanium oxide coated mica (mica powder coated with titanium oxide), and bismuth oxychloride; and metallic powder pigments such as aluminum powder and copper powder.

Examples of the dyes include: organic pigments such as red color No. 201, red color No. 202, red color No. 204, red color No. 205, red color No. 220, red color No. 226, red color No. 228, red color No. 405, orange color No. 203, orange color No. 204, yellow color No. 205, yellow color No. 401, and blue color No. 404; organic pigments such as zirconium, barium and aluminum lakes of red color No. 3, red color No. 104, red color No. 106, red color No. 227, red color No. 230, red color No. 401, red color No. 505, orange color No. 205, yellow color No. 4, yellow color No. 5, yellow color No. 202, yellow color No. 203, green color No. 3, and blue color No. 1; and natural dyes such as chlorophyll and beta-carotene.

Examples of the oil components include hydrocarbons such as squalane, liquid paraffin, Vaseline, microcrystalline wax, ozokerite, ceresin, myristic acid, palmitic acid, stearic acid, oleic acid, isostearic acid, cetyl alcohol, hexadecyl alcohol, oleyl alcohol, cetyl-2-ethylhexanoate, 2-ethylhexyl palmitate, 2-octyldodecyl myristate, neopentyl glycol di-2-ethylhexanoate, glycerol tri-2-ethylhexanoate, 2-octyldodecyl oleate, isopropyl myristate, glycerol triisostearate, glycerol tri-coconut oil fatty acid, olive oil, avocado oil, yellow beeswax, myristyl myristate, mink oil, and lanoline; silicone oil; higher fatty acid; esters of oils and fats; higher alcohol; and wax.

Further, examples of the other components include: organic solvents such as acetone, toluene, butyl acetate, and acetate ester; resins such as alkyd resin and urea resin; plasticizers such as camphor and acetyl tributyl citrate; ultraviolet absorbers; antioxidants; preservatives; surfactants; moisturizers; fragrances; water; alcohol; and thickeners.

There are various forms of cosmetics. Examples thereof include powder, cake, pencil, stick, ointment, liquid, emulsion, and cream forms. Examples of these cosmetics include: facial skin care cosmetics such as skin lotion, skin milk, and skin cream; and makeup cosmetics such as foundation, lipstick, eye shadow, cheek rouge, eyeliner, nail enamel, and mascara.

### EXAMPLES

Hereafter, the present invention is described in further detail with reference to Examples.

### (Examples 1-4)

In Examples 1 to 4, glass flakes to be mixed in the cosmetic were made of E glass or boron-free glass. More specifically, the glass flakes of Examples 1 to 4 were made using Type 1 glass or Type 2 glass having compositions shown in Table 2.

**Table 2**

| (Unit: mass%) | | | |
|---|---|---|---|
| Component | Type 1 glass | Type 2 glass | Type 3 glass |
| SiO₂ | 54.7 | 61.1 | 67.5 |
| Al₂O₃ | 14.0 | 11.2 | 4.1 |
| CaO | 23.4 | 21.7 | 6.5 |
| MgO | 0.3 | 3.1 | 2.6 |
| Na₂O | 0.4 | 1.3 | 9.4 |
| K₂O | 0.2 | 0.6 | 1.5 |
| Li₂O | - | 0.6 | 0.6 |
| B₂O₃ | 5.8 | - | 4.5 |
| ZnO | - | - | 3.2 |
| Others | 1.2 | 0.4 | 0.1 |

By using these glass compositions, the glass flakes of Examples 1 to 4 each were obtained by a blowing method as described below.

First, glass having a Type 1 glass composition or a Type 2 glass composition is put into a melting tank that has been heated to 1200°C or higher so as to melt the glass. Next, a nozzle is put in the melting tank, the molten glass is inflated with air fed through the nozzle to be formed into a thin glass film, and the thin glass film is continuously drawn out of the tank by rollers. By changing the amount of air to be fed and the rotational speed of the rollers, glass films with average thicknesses of 0.4 µm and 0.7 µm were obtained respectively. After that, the glass films were pulverized and classified, and thus glass flakes having an average particle diameter of 20 µm were obtained. The average particle diameter was measured with a laser diffraction particle size distribution analyzer (Product name "Microtrack (registered trademark) HRA" manufactured by Nikkiso Co., Ltd.).

The pulverization was carried out using a jet mill type pulverizer (Product name "Labojet LJ" manufactured by Nippon Pneumatic Mfg. Co., Ltd.). The particle size was adjusted by sieve classification. The sieve classification was carried out using an electromagnetic sieve shaker (Product name "RETSCH SIEVE SHAKER, type VIBRO" manufactured by RETSCH Co., Ltd.), and a sieve was selected appropriately for each particle size distribution to be obtained.

Table 3 shows the particle diameter distribution index (an index obtained by dividing D90 by D10) of the glass flakes. The definitions of D10 and D90 are as described above. The particle size distribution was measured here with a laser diffraction particle size distribution analyzer (Product name "Microtrack (registered trademark) HRA" manufactured by Nikkiso Co., Ltd.). D90 and D10 were obtained based on the measurement results so as to calculate the particle diameter distribution index.

Table 3 shows the glass compositions, average thicknesses and particle diameter distribution indices of the glass flakes of Examples 1 to 4.

### (Comparative Example 1)

Glass flakes of Comparative Example 1 were produced in the same manner as in Example 1 except that the average thickness and particle diameter distribution index were different from those of Example 1. The glass composition, average thickness and particle diameter distribution index of the glass flakes of Comparative Example 1 are as shown in Table 3.

### (Comparative Example 2)

Glass flakes of Comparative Example 2 were produced in the same manner as in Example 1 except that Type 3 glass composition as shown in Table 2 was used and that the average thickness and particle diameter distribution index were different from those of Example 1. The glass composition, average thickness and particle diameter distribution index of the glass flakes of Comparative Example 2 are as shown in Table 3.

### (Comparative Example 3)

In Comparative Example 3, commercially available natural mica for cosmetics (Product name "Y-2400" manufactured by Yamaguchi Mica Industry Co., Ltd.) was used. The particle diameter distribution index of the natural mica used in Comparative Example 3 is as shown in Table 3.

### (Comparative Example 4)

In Comparative Example 4, commercially available synthetic mica (Product name "PDM-9WA" manufactured by Topy Industries Ltd.) was used. The particle diameter distribution index of the synthetic mica used in Comparative Example 4 is as shown in Table 3.

**Table 3**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Glass, Mica | Type 1 glass | Type 1 glass | Type 2 glass | Type 2 glass | Type 1 glass | Type 3 glass | Natural mica | Synthetic mica |
| Alkali metal oxides in glass (mass%) | 0.6 | 0.6 | 2.5 | 2.5 | 0.6 | 11.5 | - | - |
| Average thickness (µm) | 0.7 | 0.4 | 0.7 | 0.4 | 1.3 | 1.3 | 0.3 | 0.3 |
| Average aspect ratio | 28.6 | 50 | 28.6 | 50 | 15.4 | 15.4 | 56.7 | 43.3 |
| Particle diameter distribution index | 3.8 | 3.5 | 4.0 | 3.7 | 4.1 | 3.9 | 5.6 | 5.3 |
| Transparency (ΔL value) | 3.4 | 4.2 | 3.4 | 3.9 | 4.2 | 3.4 | 10.8 | 6.5 |
| Whiteness | 68.1 | 68.3 | 68.2 | 68.5 | 69.2 | 70.1 | 56.1 | 70.4 |
| Yellowness | 4.0 | 4.5 | 3.9 | 4.2 | 3.8 | 3.5 | 28.8 | 6.5 |
| pH | 8.8 | 9.0 | 9.1 | 9.3 | 8.7 | 10.4 | 8.8 | 9.2 |

The transparency, whiteness, yellowness and pH of the glass flakes and mica of Examples 1 to 4 and Comparative Examples 1 to 4 were measured respectively. Table 3 shows the measurement results.

9 g of acrylic resin (including 40% of solid content) was added to 1 g of glass flakes or mica of each of Examples 1 to 4 and Comparative Examples 1 to 4, and the resultant mixture was stirred and mixed well. Then, the mixture was applied to a hiding power test paper using an applicator with a gap of 9 mil (9/1000 x 25.4 mm), and dried. Thus, a test panel was obtained. The hue (L value) of the black area of the test panel was measured using a colorimeter (CR-400 manufactured by Minolta Co., Ltd.). Meanwhile, a test panel for comparison was prepared by applying only acrylic resin to a test paper by the same method. The hue (L value) of the black area of this test panel for comparison was also measured in the same manner. Thus, for each of Examples 1 to 4 and Comparative Examples 1 to 4, the difference between the L values (ΔL) resulted from whether or not the glass flakes or mica were contained was obtained. It was evaluated that a smaller ΔL value indicated a higher transparency.

Furthermore, liquid paraffin was added to the glass flakes or mica of each of Examples 1 to 4 and Comparative Examples 1 to 4 in the proportion of 2 liquid paraffin to 1 glass flakes or mica so as to be formed into a paste. The whiteness and yellowness of this paste also were measured. The whiteness and yellowness were measured using "Color Meter ZE2000" (manufactured by Nippon Denshoku Industries Co., Ltd.).

Moreover, 90 g of ion exchanged water was added to 10 g of the glass flakes or mica of each of Examples 1 to 4 and Comparative Examples 1 to 4, and the resultant mixture was stirred, and then maintained under an atmosphere of 40°C. After 24 hours, the pH was measured.

In Examples 1 to 4 and Comparative Examples 1 and 2, glass flakes were obtained by a blowing method, but a rotary method may be used. In the rotary method, glass flakes having the same average thickness as described above may be obtained by changing the glass melting temperature and the rotational speed of a rotary, followed by pulverization and classification.

The measurement results showed that the glass flakes of Comparative Example 2, made using Type 3 glass with a high content of alkali metal oxides, had a higher value of pH than that of each of Examples 1 to 4 and Comparative Example 1 made using Type 1 or Type 2 glass. The natural mica of Comparative Example 3 had a lower transparency and a higher yellowness than the glass flakes of each of Examples 1 to 4 and Comparative Examples 1 and 2. The synthetic mica of Comparative Example 4 had a relatively high yellowness.

### (Examples 5-8 and Comparative Examples 5-8)

In order to evaluate as cosmetics, powder foundations were prepared using, as extender pigments, samples of Examples 1 to 4 and Comparative Examples 1 to 4, as well as respective components shown in Table 4 below. The powder foundations prepared using the glass flakes of Examples 1 to 4 are Examples 5 to 8 respectively, and those prepared using the glass flakes or mica of Comparative Examples 1 to 4 are Comparative Examples 5 to 8 respectively.

**Table 4**

| | Component | Parts by mass |
|---|---|---|
| (1) | Glass flakes of Examples 1 to 4, or glass flakes or mica of Comparative Examples 1 to 4 | 35 parts by mass |
| (2) | Talc | 20 parts by mass |
| (3) | Glass flakes coated with titanium | 20 parts by mass |
| (4) | Titanium dioxide | 10 parts by mass |
| (5) | Spherical polyethylene powder | 5 parts by mass |
| (6) | Colcothar | 1.0 parts by mass |
| (7) | Yellow iron oxide | 3.0 parts by mass |
| (8) | Black iron oxide | 0.1 parts by mass |
| (9) | Silicone oil | 1 parts by mass |
| (10) | 2-ethylhexyl palmitate | 9 parts by mass |
| (11) | Sorbitan sesquioleate | 1 parts by mass |
| (12) | Preservative | 0.3 parts by mass |
| (13) | Fragrance | 0.1 parts by mass |

The components (1) to (8) were mixed by a Henschel mixer. To this mixture, the components (9) to (13) dissolved and mixed by heat were added and then pulverized by a pulverizer. The resultant powder was molded into a plate of 6 cm in diameter at a pressure of 1.47 × 10⁻¹MPa (1.5 kg/cm²). Thus, the powder foundations of respective Examples and Comparative Examples were obtained.

For the powder foundations prepared in Examples 5 to 8 and Comparative Examples 5 to 8, a sensory evaluation test was carried out.

For the sensory evaluation test, 10 panelists were employed. The feeling of use of the cosmetics was evaluated based on the average values in the evaluation by the 10 panelists. Table 5 shows the evaluation standards, and Table 6 shows the evaluation results. The feeling after washing was evaluated based on the degree of fresh and clean feeling after washing the sample powder foundations put on the face with cleansing soap and water.

**Table 5**

| Evaluation point | Finish | Transparency | Feeling of use (spreading) | Feeling after washing |
|---|---|---|---|---|
| 1 | Very unnatural | None | Very bad | Very bad |
| 2 | Unnatural | Low | Bad | Bad |
| 3 | Middling | Middling | Middling | Middling |
| 4 | Acceptably natural | Fairly high | Fairly good | Fairly good |
| 5 | Natural | High | Excellent | Excellent |

**Table 6**

| | Example 5 | Example 6 | Example 7 | Example 8 | Comparative Example 5 | Comparative Example 6 | Comparative Example 7 | Comparative Example 8 |
|---|---|---|---|---|---|---|---|---|
| Glass flakes or mica used | Example 1 | Example 2 | Example 3 | Example 4 | Comparative Example 1 | Comparative Example 2 | Comparative Example 3 | Comparative Example 4 |
| Finish | 5 | 5 | 5 | 5 | 4.3 | 3.9 | 3.5 | 4.6 |
| Transparency | 5 | 5 | 5 | 5 | 5 | 5 | 2.3 | 4.1 |
| Feeling of use (spreading) | 4.2 | 5 | 4.4 | 5 | 2.3 | 2.6 | 5 | 5 |
| Feeling after washing | 4.2 | 4.8 | 4.5 | 4.6 | 5 | 5 | 4.1 | 4.2 |

As described above, the samples (Examples 5 to 8) using the glass flakes in the cosmetic of the present invention as a base material for the powder foundations showed excellent results in the finish, transparency, and feeling after washing, compared to the products (Comparative Examples 7 and 8) using the conventional natural mica and synthetic mica as base materials.

In addition, the products (Examples 5 to 8) using the glass flakes in the cosmetic of the present invention as a base material showed excellent results in the finish and feeling of use (spreading), compared to the products of Comparative Examples 5 and 6 containing glass flakes with a greater average thickness. It should be noted that the glass flakes in the cosmetic of the present invention are glass flakes that satisfy the following conditions: a composition including at least 52 mass% of silicon dioxide and 0 to 5 mass% of alkali metal oxides; an average thickness of 0.1 to 1.0 µm; an average particle diameter of 1 to 100 µm; an average aspect ratio of 10 or greater; and a particle diameter distribution index of 5.0 or less.

### Industrial Applicability

The cosmetic of the present invention can be used as a makeup cosmetic that is smooth and good to the touch, is excellent in transparency and adhesion to the skin, and exhibits a natural finish with moderate gloss, so-called a natural skin finish.

## Claims

1. A cosmetic containing glass flakes,
- wherein the glass flakes have the following composition of E glass or Boron-free glass in mass%:
| Component | E glass | Boron-free E glass |
|---|---|---|
| SiO₂ | 52 to 56 | 55 to 67 |
| Al₂O₃ | 12 to 16 | 5 to 15 |
| CaO | 16 to 25 | 17 to 25 |
| MgO | 0 to 6 | 0 to 6 |
| Na₂O+K₂O | 0 to 0.8 | 0 to 5 |
| B₂O₃ | 5 to 13 | - |
| TiO₂ | - | 0 to 2 |
| F₂ | 0 to 0.5 | - |
- and the glass flakes have an average thickness of 0.1 to 1.0 µm, an average particle diameter of 1 to 100 µm, and an average aspect ratio of 10 or greater, the average aspect ratio being obtained by dividing the average particle diameter by the average thickness, and
- a particle diameter distribution index of the glass flakes is 5.0 or less, the particle diameter distribution index being obtained by dividing D90 by D10, where in a particle size distribution of the glass flakes, D10 is a particle diameter at which the cumulative mass distribution of particle diameters reaches 10% when counted from the smaller side, and D90 is a particle diameter at which the cumulative mass distribution of particle diameters reaches 90% when counted from the smaller side,
wherein at least part of the surface of each of the glass flakes is coated with a hydrophobizing agent.

2. The cosmetic according to Claim 1,
wherein the particle diameter distribution index is 4.0 or less.

3. The cosmetic according to Claim 1,
wherein the glass flakes have a composition including 3 mass% or less of alkali metal oxides.

4. The cosmetic according to Claim 1,
wherein the glass flakes are mixed as an extender pigment in the cosmetic in an amount of 5 to 80 mass% of the total amount of extender pigments.

## Patentansprüche

1. Kosmetikum, umfassend Glasschuppen,
- wobei die Glasschuppen die folgende Zusammensetzung aus E-Glas oder borfreiem Glas in Massen-% aufweisen:
| Komponente | E-Glas | borfreies E-Glas |
|---|---|---|
| SiO₂ | 52 bis 56 | 55 bis 67 |
| Al₂O₃ | 12 bis 16 | 5 bis 15 |
| CaO | 16 bis 25 | 17 bis 25 |
| MgO | 0 bis 6 | 0 bis 6 |
| Na₂O+K₂O | 0 bis 0,8 | 0 bis 5 |
| B₂O₃ | 5 bis 13 | - |
| TiO₂ | - | 0 bis 2 |
| F₂ | 0 bis 0,5 | - |
- und die Glasschuppen eine durchschnittliche Dicke von 0,1 bis 1,0 µm, einen durchschnittlichen Teilchendurchmesser von 1 bis 100 µm und ein durchschnittliches Aspektverhältnis von 10 oder mehr aufweisen, wobei das durchschnittliche Aspektverhältnis durch Teilen der durchschnittlichen Teilchengröße mit der durchschnittlichen Dicke erhalten wird, und
- ein Teilchendurchmesserverteilungsindex der Glasschuppen 5,0 oder weniger beträgt, wobei der Teilchendurchmesserverteilungsindex durch Teilen von D90 mit D10 erhalten wird, wobei in einer Teilchengrößenverteilung der Glasschuppen D10 ein Teilchendurchmesser ist, bei welchem die kumulative Massenverteilung der Teilchendurchmesser 10 % erreicht, wenn sie von der kleineren Seite gezählt werden, und D90 ein Teilchendurchmesser ist, bei welchem die kumulative Massenverteilung der Teilchendurchmesser 90 % erreicht, wenn sie von der kleineren Seite gezählt werden,
wobei wenigstens ein Teil der Oberfläche von jeder Glasschuppe mit einem hydrophobisierenden Wirkstoff beschichtet ist.

2. Kosmetikum nach Anspruch 1,
wobei der Teilchendurchmesserverteilungsindex 4,0 oder weniger beträgt.

3. Kosmetikum nach Anspruch 1,
wobei die Glasschuppen eine Zusammensetzung aufweisen, die 3 Massen-% oder weniger eines Alkalimetalloxides umfassen.

4. Kosmetikum nach Anspruch 1,
wobei die Glasschuppen als ein Füllstoffpigment in das Kosmetikum in einer Menge von 5 bis 80 Massen-% der Gesamtmenge von Füllstoffpigmenten gemischt sind.

## Revendications

1. Cosmétique contenant des lamelles de verre,
- où les lamelles de verre ont la composition suivante de verre E ou de verre sans bore en % en masse :
| Composant | Verre E | Verre E sans bore |
|---|---|---|
| SiO₂ | 52 à 56 | 55 à 67 |
| Al₂O₃ | 12 à 16 | 5 à 15 |
| CaO | 16 à 25 | 17 à 25 |
| MgO | 0 à 6 | 0 à 6 |
| Na₂O+K₂O | 0 à 0,8 | 0 à 5 |
| B₂O₃ | 5 à 13 | - |
| TiO₂ | - | 0 à 2 |
| F₂ | 0 à 0,5 | - |
- et les lamelles de verre ont une épaisseur moyenne de 0,1 à 1,0 µm, un diamètre de particule moyen de 1 à 100 µm, et un rapport d'aspect moyen de 10 ou plus, le rapport d'aspect moyen étant obtenu en divisant le diamètre de particule moyen par l'épaisseur moyenne, et
- un indice de distribution des diamètres de particule des lamelles de verre est 5,0 ou moins, l'indice de distribution des diamètres de particule étant obtenu en divisant D90 par D10, où, dans une distribution de tailles de particule des lamelles de verre, D10 est un diamètre de particule auquel la distribution en masse cumulée des diamètres de particule atteint 10 % quand ils sont comptés depuis le plus petit côté, et D90 est un diamètre de particule auquel la distribution en masse cumulée des diamètres de particule atteint 90 % quand ils sont comptés depuis le plus petit côté,
où au moins une partie de la surface de chacune des lamelles de verre est recouverte d'un agent hydrophobant.

2. Cosmétique selon la revendication 1, où l'indice de distribution des diamètres de particule est 4,0 ou moins.

3. Cosmétique selon la revendication 1 où les lamelles de verre ont une composition incluant 3 % en masse ou moins d'oxydes de métaux alcalins.

4. Cosmétique selon la revendication 1, où les lamelles de verre sont mélangées sous forme d'un pigment extendeur dans le cosmétique en une quantité de 5 à 80 % en masse de la quantité totale de pigments extendeurs.
